# EUROPEAN PATENT APPLICATION

(11) **EP 3 202 416 A1**
(43) Date of publication of application: **09.08.2017**
(21) Application number: 17162197.2
(22) Date of filing: 21.04.2011
(51) Int. Cl.: A61K 38/40, A61K 39/42, A61P 31/04, A61P 31/12

(54) **COMPOSITION COMPRISING LACTOFERRIN AND IMMUNOGLOBULIN**

(30) Priority: 23.04.2010 AU 2010901715
(62) Divisional of application: 11771411.3
(71) Applicant: Probiotec Limited, Laverton North, Victoria 3026 (AU)
(72) Inventor: GANTER, Rudi, Victoria, 3026 (AU); AHMAD, Humera, Victoria, 3026 (AU)
(74) Representative: Lee, Nicholas John

(57) **Abstract**

A composition including lactoferrin and immunoglobulin, wherein the composition does not substantially include one or more of the following proteins: lactoperoxidase, lactoglobulin, albumin.

## Description

### Field of the invention

The invention relates to compositions including lactoferrin and immunoglobulin, to processes for production of same and to uses thereof for the treatment of conditions and diseases.

Reference to any prior art in the specification is not, and should not be taken as, an acknowledgment or any form of suggestion that this prior art forms part of the common general knowledge in Australia or any other jurisdiction or that this prior art could reasonably be expected to be ascertained, understood and regarded as relevant by a person skilled in the art.

It will be understood that the invention disclosed and defined in this specification extends to all alternative combinations of two or more of the individual features mentioned or evident from the text or drawings. All of these different combinations constitute various alternative aspects of the invention.

### Summary of the invention

In certain embodiments there is provided a composition including lactoferrin and immunoglobulin, wherein the composition does not substantially include one or more of the following proteins: lactoperoxidase, lactoglobulin, albumin, lactalbumin.

In another embodiment there is provided a composition consisting substantially of lactoferrin and immunoglobulin.

In another embodiment there is provided a composition including lactoferrin and immunoglobulin in synergistically effective amounts.

In another embodiment there is provided a pharmaceutical or therapeutic composition for minimising the severity of one or more symptoms associated with common cold including lactoferrin and immunoglobulin in therapeutically effective amounts.

In one embodiment the composition is produced by a process including the steps of:
-providing a source of lactoferrin;
providing a source of immunoglobulin;
combining said source of lactoferrin with said source of immunoglobulin, thereby producing the composition, wherein, the source of immunoglobulin includes immunoglobulin in an amount of 30 to 95 (w/w%) of the source.

In another embodiment there is provided a use of a composition described above for minimizing the severity of one or more symptoms associated with common cold.

In another embodiment there is provided a use of a composition described above in the manufacture of a medicament for minimizing the severity of one or more symptoms associated with common cold.

In another embodiment there is provided a method of minimising the severity of one or more symptoms associated with common cold including the step of administering a composition described above to an individual having a common cold.

### Description of the invention

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

As used herein, except where the context requires otherwise, the term *"comprise"* and variations of the term, such as *"comprising", "comprises"* and *"comprised",* are not intended to exclude further additives, components, integers or steps.

As used herein, the phrase *'wherein the composition does not substantially include'* generally refers to a substantial, but not complete absence of a specified reagent, component or compound in the composition the subject of the phrase, specifically that the specified reagent, component or compound may be present in the composition at most in residual or trace amounts, or amounts that do not substantially influence the activity of the lactoferrin and/or immunoglobulin for minimizing one or more symptoms of a condition of concern, such as a common cold. In one example, the residual or trace amounts of the specified reagent, component or compound in the composition may arise from unintended contamination of the composition or from imperfect separation of lactoferrin or immunoglobulin from a precursor material used in the preparation of the composition, one example of such a material being whey.

As used herein, the phrase *'wherein the composition consists substantially of'* generally refers to a composition in which the components having the higher relative abundance or higher weight as a percentage of total weight of the composition (except for diluents, excipients, fillers and the like) are lactoferrin and/or immunoglobulin.

As used herein, *'active ingredient'* generally refers to an ingredient having activity for the treatment of a disease or condition, for example for reducing or minimising the severity of one or more symptoms associated with common cold. Lactoferrin and immunoglobulin are active ingredients of the composition according to the invention.

As used herein, *'pharmaceutically acceptable'* generally refers to a substance or composition that is compatible chemically and/or toxicologically, with the other components included in a composition, and/or the mammal being treated therewith.

As used herein, *'synergy'* generally refers to a relationship between 2 or more components whereby when combined for use, the combined effect of the 2 or more components is quantitatively and/or qualitatively different to the sum effect arising from individual use of each component.

As used herein, *'synergistically effective amount'* generally refers to an amount of each component required to provide synergy with other components.

As used herein, *'common cold'* generally refers to a condition characterised by symptoms that are commonly observed in an infection caused by rhinovirus and/or coronavirus. The symptoms may include one or more of the following: sore throat, nasal congestion, nasal discharge, cough, sneezing, mild fatigue, mild headaches and possible low grade fever.

As used herein *'symptoms associated with common cold'* generally refers to sore throat, nasal congestion, nasal discharge, cough, sneezing, mild fatigue, mild headaches and possible low grade fever.

As used herein, *'minimizing the severity of symptoms associated with common cold'* is intended to mean a reduction of the duration of one or more symptoms associated with common cold, and/or a reduction in the number of symptoms associated with common cold and/ or a reduction in the extent of one or more symptoms associated with common cold. For example, the duration of common cold symptoms may be reduced from 3 to 5 days to just 2 to 3 days, or the nasal discharge may lessen.

As used herein, *'therapeutically effective amount'* generally refers to an amount of a composition of the present invention that (i) treats the particular disease, condition, or disorder, (ii) attenuates, ameliorates, or eliminates one or more symptoms of the particular disease, condition, or disorder, or (iii) delays the onset of one or more symptoms of the particular disease, condition, or disorder described herein.

As used herein, the words *'treat'* or *'treatment'* generally refer to therapeutic treatment wherein the object is to slow down (lessen) an undesired physiological change or disorder. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Treatment may not necessarily result in the complete clearance of an infection but may reduce or minimise complications and side effects of infection and the progression of infection.

As used herein, the words *'prevent'* and *'prevention'* refer to prophylactic or preventative measures for protecting or precluding an individual not having a given infection related complication from progressing to that complication. Individuals in which prevention is required include those who have an infection.

*'Lactoferrin'* (LF) also known as lactotransferrin (LTF), is a globular multifunctional protein that can be obtained from whey or related dairy products, or from recombinant technology. Lactoferrin may have a high affinity for ferrous and/or ferric ions. Lactoferrin proteolysis also produces the small peptides lactoferricin and kaliocin-1. These peptides and other lactoferrin -related peptides may, in certain embodiments, be used in addition, or in alternative to lactoferrin.

'Immunoglobulin' or 'antibody' or *'lg'* are gamma globulin proteins that are found in milk, blood, or other bodily fluids of verterbrates that function in the immune system to bind antigen, hence identifying and neutralizing foreign objects.

Antibodies are generally a heterotetrameric glycoprotein composed of two identical light (L) chains and two identical heavy (H) chains. Each L chain is linked to a H chain by one covalent disulfide bond. The two H chains are linked to each other by one or more disulfide bonds depending on the H chain isotype. Each H and L chain also has regularly spaced intrachain disulfide bridges.

H and L chains define specific lg domains. More particularly, each H chain has at the N-terminus, a variable domain (V_{H}) followed by three constant domains (C_{H}) for each of the α and γ chains and four C_{H} domains for µ and ε isotypes. Each L chain has at the N-terminus, a variable domain (V _{L}) followed by a constant domain (C_{L}) at its other end. The V_{L} is aligned with the V_{H} and the C_{L} is aligned with the first constant domain of the heavy chain (C_{H}1).

Antibodies can be assigned to different classes or isotypes. There are five classes of immunoglobulins: IgA, IgD, IgE, IgG, and IgM, having heavy chains designated α, δ, ε, γ, and µ, respectively. The γ and α classes are further divided into subclasses on the basis of relatively minor differences in C_{H} sequence and function, e.g., humans express the following subclasses: IgG1, IgG2, IgG3, IgG4, IgA1, and IgA2. The L chain from any vertebrate species can be assigned to one of two clearly distinct types, called kappa and lambda, based on the amino acid sequences of their constant domains.

In certain embodiments there is provided a composition including lactoferrin and immunoglobulin, wherein the composition does not substantially include one or more of the following proteins: lactoperoxidase, lactoglobulin, albumin, lactalbumin.

Preferably the composition includes lactoferrin and immunoglobulin and does not substantially include lactoperoxidase. In this form, the composition may include lactoglobulin and albumin.

Preferably the composition includes lactoferrin and immunoglobulin and does not substantially include lactoglobulin. In this form, the composition may include lactoperoxidase and albumin.

Preferably the composition includes lactoferrin and immunoglobulin and does not substantially include albumin. In this form, the composition may include lactoperoxidase and lactoglobulin.

Preferably the composition includes lactoferrin and immunoglobulin and does not substantially include lactoglobulin or albumin. In this form, the composition may include lactoperoxidase.

Preferably the composition includes lactoferrin and immunoglobulin and does not substantially include lactoperoxidase or albumin. In this form, the composition may include lactoglobulin.

Preferably the composition includes lactoferrin and immunoglobulin and does not substantially include lactoperoxidase or lactoglobulin. In this form, the composition may include albumin.

In another embodiment there is provided a composition consisting substantially of lactoferrin and immunoglobulin.

In one embodiment, the lactoferrin and immunoglobulin may be present in the composition in amounts of a 2:1 ratio of lactoferrin to immunoglobulin, i.e. g/g or mass:mass ratio, however, lesser amounts of lactoferrin relative to immunoglobulin tending towards a 1:1 ratio are also contemplated, as are greater amounts of lactoferrin, for example to 5:1 ratio of lactoferrin to immunoglobulin.

In one embodiment where the composition is provided in a form of a capsule, enteric or film coated caplet, enteric or film coated tablet or like, immunoglobulin is provided in an amount of 1 to 98 (w/w%) of the capsule, caplet, tablet or like. The lactoferrin may be provided in an amount of 1 to 98 (w/w%) of the capsule, caplet, tablet or like.

In one embodiment where the composition is provided in the form a liquid, the immunoglobulin is provided in an amount of 0.1- 25 (w/w%) of the liquid. The lactoferrin may be provided in an amount of 0.1- 25 (w/w%) of the liquid.

In one embodiment where the composition is provided in a form for topical application, the immunoglobulin is provided in an amount of 0.1- 25 (w/w%) of the composition. The lactoferrin is provided in an amount of 0.1- 25 (w/w%) of the composition.

In another embodiment there is provided a composition including lactoferrin and immunoglobulin in synergistically effective amounts.

In yet another embodiment there is provided a pharmaceutical or therapeutic composition for minimising the severity of one or more symptoms associated with common cold including lactoferrin and immunoglobulin in therapeutically effective amounts.

In another embodiment the composition described above is provided in a form suitable for oral administration.

Preferably the composition is provided in the form of a capsule, enteric or film coated caplet, enteric or film coated tablet, powder sachet or like. In one embodiment, the immunoglobulin is provided in an amount of 1 to 98 (w/w%), preferably 1 to 50 (w/w%), preferably 50 to 98 (w/w%), still more preferably about 10 to 30 (w/w%), still more preferably about 16% of the capsule, caplet, tablet or like.

Further, where the composition is provided in the form of a capsule, caplet, tablet or like the lactoferrin is provided in an amount of 1 to 98 (w/w%), more preferably 1 to 50 (w/w%), still more preferably 50 to 98 (w/w%), still more preferably about 50 to 70 (w/w%), still more preferably about 66% of the capsule, caplet, tablet or like.

Further, where the composition is provided in the form of a capsule, caplet, tablet or like, an excipient may be provided in an amount of 1 to 5 (w/w%) of the capsule, caplet, tablet or like. Any excipient for minimising clumping or otherwise for improving dispersal of immunoglobulin and lactoferrin may be used, such as a stearate, preferably, magnesium stearate. Other excipients are well known in the art, examples including microcrystalline cellulose, silicon dioxide, colloidal silicon and microcrystalline cellulose, leucine and starch.

The composition may be entrapped in soft gel capsules. It may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Caplets, capsules, tablets and the like may be enteric or gel coated.

The composition may be provided in the form of slow released, delayed release or sustained release formulations. Capsules, tablets, caplets and the like may contain an enteric coating.

It may be beneficial for the composition of the invention to include a further active ingredient. These may be incorporated into the composition, depending on the anticipated route of administration and the stage of the infection or related complications. For example, the therapeutic composition may further include an anti-histamine, an analgesic, a decongestant, an expectorant, or a cough suppressant.

In another embodiment, the further active ingredient is a probiotic, such as a gram positive strain of bacteria. Other particularly useful bacteria may be Lactobacillus paracasei subsp. Paracasei, Lactobacillus acidophilus, Lactobacillus rhamnosus, Lactobacillus reuteri, Streptococcus thermophilus and Bifidobacterium lactis.

In the case of co-infections or secondary infections, particularly those arising from complications of infection, the therapeutic composition may include one or more antiviral, anti-bacterial, anti-fungal and anti-protozoan agents.
In other embodiments the composition does not contain a further active ingredient.

The composition may also be provided in the form of a liquid such as a syrup or the like. In one embodiment, the immunoglobulin is provided in an amount of 0.1 to 25 (w/w%) of the liquid, more preferably 10 to 25 (w/w%), still more preferably 1 to 10 (w/w%), still more preferably 0.5 to 1.5 (w/w%), more preferably about 1.1 (w/w%).

Further, where the composition is provided in the form of a liquid, the lactoferrin may be provided in an amount of 0.1 to 25 (w/w%) of the liquid, more preferably 10 to 25 (w/w%), more preferably 1 to 10 (w/w%), still more preferably 1.0 to 3.0 (w/w%), more preferably about 2.5 (w/w%).

Further, where the composition is provided in the form of a liquid, a solvent for the lactoferrin and/or immunoglobulin may be provided. One example of a solvent is water.

Further, where the composition is provided in the form of a liquid, one or more of the following compounds or components may be provided: antimicrobial compounds, flavours, thickeners etc.

As detailed above in relation to a capsule formulation of the composition of the invention, it may be beneficial for the composition to include a further active ingredient. These may be incorporated into the composition, depending on the anticipated route of administration and the stage of the infection or related complications.

In other embodiments the composition does not contain a further active ingredient.

In another embodiment the composition is provided in a form suitable for topical administration, especially to skin, mucosa or other body surface.

Preferably the composition is provided in the form of a gel, cream, paste or spray suitable for nasal or skin application. For application to mucosal surfaces such as the eye, the composition is provided in the form of a liquid able to be administered as eye drops.

In one embodiment, the composition is provided in the form of a gel and the immunoglobulin is provided in an amount of 0.1 to 25 (w/w%) of the liquid, more preferably 10 to 25 (w/w%), still more preferably 1 to 10 (w/w%), still more preferably 5 to 10 (w/w%), more preferably about 5.0 (w/w%).

Further, where the composition is provided in the form of a gel, the lactoferrin may be provided in an amount of 0.1 to 25 (w/w%) of the liquid, preferably 10 to 25 (w/w%), more preferably 1 to 15 (w/w%), still more preferably 10 to 15 (w/w%), more preferably about 10 (w/w%).

Further, where the composition is provided in the form of a gel, an emollient or penetrant may be provided. One example of an emollient is glycerol.

A topical formulation of the composition of the invention, may also include a further active ingredient, such as those already listed above and including one or more of an anti-histamine or an analgesic, or a probiotic. Other active ingredients may include hyaluronic acid, glycosylaminoglycan (GAGs) and corticosteroids.

In other embodiments the composition does not contain a further active ingredient.

Pastes and creams may, for example, be formulated with an aqueous or oily base with the addition of suitable thickening and/or gelling agents. Lotions and gels may be formulated with an aqueous or oily base, and will in general also contain one or more emulsifying agents, stabilising agents, dispersing agents, suspending agents, thickening agents, or colouring agents.

In certain embodiments, formulations for topical administration may include one or more of the following ingredients together with the therapeutically effective amount of lactoferrin and immunogloblulin: sodium chloride, L-histidine, sodium borate, lactic acid, sodium phosphate monobasic, sorbitan monostearate, polysorbate 60, cetyl esters wax, benzyl alcohol, glycerol, cetostearyl alcohol, isopropyl myristate, propylene glycol, purified water, chlorohexidine hydrochloride octyldodecanol, sodium hydroxide, stearic acid and paraffin liquid.

In any of the above types of formulations, the composition may further comprises a pharmaceutically acceptable diluent, carrier, excipient or like compound. Generally, these components are ones that are added to the composition in the production or formulation of it. Acceptable diluents, carriers, excipients, and stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins such as gelatin; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The different formulations of the composition of the invention may be packaged in a variety of ways depending upon the method used for administering the drug. Generally, a kit or article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. Foil and/or PVC blister packs are particularly useful. The container may also include a tamper-proof assemblage to prevent indiscreet access to the contents of the package. In addition, the container has deposited thereon a label that describes the contents of the container. The label may also include appropriate warnings.

It is especially advantageous to formulate the compositions of the present invention in unit dosage form for ease of administration and uniformity of dosage. The specifications for the dosage unit forms of the present invention may be determined by a person skilled in the art together with the information provided below, depending on, for example (a) the characteristics of the lactoferrin and immunoglobulin and the particular therapeutic effect to be achieved, and (b) the limitations inherent in the art of compounding such active ingredients for the particular treatment.

Generally when administered for therapy of cold, the composition may be administered as 2 capsules, enteric or film coated caplets, enteric or film coated tablets or like (as described above), or 20 mls of liquid formulation, (as described above) every 4 hours.

Where prevention is intended, the composition is administered in as 2 capsules, caplets, tablets or like (as described above), or 20 mls of liquid formulation, (as described above) daily.

Typically the dosing will be for 1 to 2 weeks where treatment is required.

Where prevention is required, the dosing may be about 30 days, 45 days, 90 days or longer.

Preferably about 50 to 2000mg, more preferably about 50 to 600mg of lactoferrin is administered per day. Preferably about 1 to 2000mg, more preferably about 50 to 600mg of immunoglobulin is administered per day.

Typically, the above described compositions are produced by a process including the steps of:
-providing a source of lactoferrin;
providing a source of immunoglobulin;
combining said source of lactoferrin with said source of immunoglobulin, thereby producing the composition. In this embodiment, the source of immunoglobulin includes immunoglobulin in an amount of 40 to 95 (w/w%) of the source, more preferably 55 to 60 (w/w%), still more preferably 45 to 50 (w/w%), still more preferably about 48 (w/w%). Preferably the immunoglobulin includes about 40 to 50 (w/w)% IgG and about 5-10 (w/w)% IgA.

The lactoferrin source may be a composition wherein the composition consists substantially of lactoferrin.

Typically the composition includes all immunoglobulin classes, namely, IgG, IgM, IgA, IgE and IgD, and sub-classes thereof, especially IgG1, IgG2, IgG3, IgG4 and IgA1 and IgA2. In certain embodiments, the composition does not substantially include one or more of IgM, IgA, IgE and IgD.

In one embodiment the immunoglobulin is not enriched for a particular class or subclass. For example, immunoglobulin is not enriched for IgA, or in another example, not enriched for IgM.

Typically at least part of the lactoferrin and immunoglobulin in the composition is obtained from a biological fluid or product derived from a biological fluid. The biological fluid may be a milk or a related dairy product. Preferably the fluid is milk, colostrum or whey stream. Preferably the related dairy product is a whey protein concentrate, isolate or related fraction.

In other embodiments the lactoferrin and immunoglobulin may be derived from a biological fluid in the form of blood, serum or the like.

Typically at least part of the immunoglobulin and/or lactoferrin in the composition is bovine, ovine, caprine, porcine or from another mammal.

In one embodiment, some or all of the lactoferrin and/or immunoglobulin in the composition may be recombinant.

In other embodiments, the composition does not contain any recombinant lactoferrin and/or recombinant immunoglobulin.

In one embodiment, the composition may include a further active ingredient. Preferably, the further active ingredient is a probiotic as described herein or a prebiotic such as inulin, oligofructose, dextrin etc.

Typically the composition does not include an enzyme.

The compositions may be prepared by uniformly and intimately bringing into association the active ingredient(s) with liquid carriers or finely divided solid carriers or both, and if necessary, shaping the product. Formulation may be conducted by mixing at ambient temperature at the appropriate pH, and at the desired degree of purity, with physiologically acceptable carriers, i.e., carriers that are non-toxic to recipients at the dosages and concentrations employed.

The pH of the formulation depends mainly on the particular use and the concentration of compound, but may range from about 3 to about 9. Formulation in an acetate buffer at pH 5 is a suitable embodiment.

The compound ordinarily will be stored as a solid composition, although lyophilized formulations or aqueous solutions are acceptable.

As exemplified in Example 1, the composition is particularly useful for minimising the severity of symptoms associated with certain diseases and conditions. Thus in one embodiment there is provided the use of a composition described above in the manufacture of a medicament for minimizing the severity of symptoms associated with common cold. In another embodiment there is provided a method of minimising the severity of symptoms associated with common cold including the step of administering a composition described above to an individual having a common cold.

### Examples

### Example 1 Clinical trial demonstrating minimisation of severity of symptoms associated with common cold.

### 1 Aims

To determine the safety and efficacy of a lactoferrin / immunoglobulin preparation to:
i) prevent the onset of a cold;
ii) reduce time of symptoms in patients with a cold.

### 2 Method

Randomized, double-blind (participants and investigators), placebo-controlled trial.

Participants were allocated one of the following treatments before breakfast for 90 days:
i) Test 600 mg/d [2x 300 mg] lactoferrin / immunoglobulin; or
ii) Placebo 600 mg/d [2x CaHP].

### 3 Study Particulars

### i) Inclusion criteria

Self report ≥ 1 colds per month;
M or F, 18 yrs or over;
Good general health;

Participants of childbearing age who agree to continue using birth control measures for the duration of the study.

### ii) Exclusion criteria

Use of vitamins, herbal preparations and probiotics or any other medications for one week prior to beginning treatment and for the duration of the study;
Use of cold and flu or Sudafed while on the study;
History of alcohol or substance abuse;
Female participants who are lactating, pregnant or planning to become pregnant;
History of serious or unstable cardiac, renal, hypertensive, pulmonary, endocrine, neurologic or neuropsychiatric disorders.
*N = 104 evaluable participants*

### iii) Outcomes

Number of symptomatic respiratory infections [SRIs] that each participant had during the study;
Total days of duration of each participant's symptoms;
Average days of duration of each participant's symptoms;
Symptom-days total for each 'cold associated' symptoms self-report daily diary;
Average symptom-day total for each participant's and distinguish between categories Colds, Flu, or Pharyngitis.

### iv) Follow-up period

Visit 1 - baseline screening;
Visit 2 - 45 days;
Visit 3 - 90 days.

### 3 Results

**i) Cold events / symptoms**

| Participant | No | 1-2 | 3-8 | Total % | Symptoms |
|---|---|---|---|---|---|
| Group | symptoms | Symptoms | Symptoms | showing symptoms | |
| Test [N=53] | 62.3% | 18.2% | 18.9% | 37.1% | cough, sneezing, sore throat, headaches, nasal congestion |
| Placebo [N=51] | 41.2% | 21.5% | 37.3% | 58.8% | cough, sneezing, sore throat, headaches, migraines, aches, fatigue, sinus congestion |

| | | | | | |
|---|---|---|---|---|---|
| *Test group v Placebo group - significantly shorter duration time of symptoms P<0.05. [2-3 days v ≥3-5 days].* | | | | | |

### ii) Adverse effects

Blood pathology - all normal FBEs / liver function tests/ urea / electrolytes
Adverse events - no serious AE recorded as due to lactoferrin / immunoglobulin preparation

### 4 Conclusions

This placebo controlled randomized trial has demonstrated:
Safety and efficacy for reducing symptomatology associated with the common cold;
Efficacy in preventing the common cold;
Reduced time to recovery.

### Example 2 Capsule formulation

An example of a formulation is as follows:

| **Ingredient** | **Range of ingredient per capsule** | **Preferred amount of ingredient per capsule** | **Preferred amounts (w/w%)** |
|---|---|---|---|
| Lactoferrin | 180-250mg | 220mg | 60-70 |
| Immunoglobulin | 90-120mg | 104mg | 25-35 |
| Magnesium stearate | 3-10mg | 5.4mg | 1-2 |

An alternative capsule formulation is:

| **Ingredient** | **Range of ingredient per capsule** | **Preferred amount of ingredient per capsule** | **Preferred amounts (w/w%)** |
|---|---|---|---|
| Lactoferrin | 50-100mg | 71.5mg | 10-20 |
| Immunoglobulin | 90-120mg | 110mg | 15-25 |
| Magnesium stearate | 10-15mg | 12.5mg | 2-3 |
| CBAR-Blend-100 | 150-200mg | 180.2mg | 30-40 |
| Inulin (Orafti GR) | 75-120mg | 96.3mg | 15-25 |
| Raftilose P95 | 7.5-12.5mg | 10.5mg | 2-3 |

### Example 3 Gel formulation

A gel formulation is as follows:

| **Ingredient** | **Range of ingredient % w/w** | **Preferred amount of ingredient % w/w** |
|---|---|---|
| Lactoferrin | 0.1-25 | 10.5 |
| Immunoglobulin | 0.1-25 | 5.0 |
| Cetomacrogol 1000 BP | 1-3 | 1.5 |
| Cetostearyl Alcohol BP | 5-10 | 6.0 |
| Paraffin - White soft BP | 2-7 | 5.0 |
| Paraffin - Liquid BP | 2-7 | 5.0 |
| Glycerol | 5-15 | 10.0 |
| Germaben II | Up to 1 | 0.60 |
| Colostrum powder | 2-7 | 5.00 |
| Water | To 100 | 51.40 |

An alternative gel formulation is as follows:

| **Ingredient** | **Range of ingredient % w/w** | **Preferred amount of ingredient % w/w** |
|---|---|---|
| Lactoferrin | 5-15 | 5.5 |
| Immunoglobulin | 1-5 | 1.0 |
| Cetomacrogol 1000 BP | 1-3 | 1.5 |
| Cetostearyl Alcohol BP | 5-10 | 6.0 |
| Paraffin - White soft BP | 2-7 | 5.0 |
| Paraffin - Liquid BP | 2-7 | 5.0 |
| Glycerol | 5-15 | 12.0 |
| Germaben II | Up to 1 | 0.60 |
| Colostrum powder | 2-7 | 5.00 |
| Water | To 100 | 61.60 |

### Example 4 Syrup formulation

A syrup (liquid) formulation may be as follows:

| **Ingredient** | **Range of ingredient % w/w** | **Preferred amount of ingredient % w/w** |
|---|---|---|
| Lactoferrin | 0.1-25 | 2.4 |
| Immunoglobulin | 0.1-25 | 1.1 |
| Sodium benzoate | 0.1-0.2 | 0.180 |
| Nipasept (preservative) | 0.05-0.1 | 0.075 |
| Povidone | 0.250-1 | 0.500 |
| Sodium carboxymethylcellulose | 0.025-0.075 | 0.050 |
| Anhydrous citric acid | 0.01-0.03 | 0.0209 |
| Monobasic potassium phosphate | Up to 0.0002 | 0.0001 |
| Glycerol | 10-15 | 20.00 |
| Strawberry flavour | Up to 0.1 | 0.050 |
| Vanilla flavour | Up to 0.1 | 0.050 |
| Amaranth | Up to 0.002 | 0.001 |
| Water | To 100 | 75.470 |

### Example 5 Process for production of immunoglobulin for use in composition

When cheese is manufactured from milk (pasteurised or unpasteurised), this results in the removal of casein, some protein and dairy fat, leaving a liquid whey stream from which lactoferrin and immunoglobulins may be isolated.

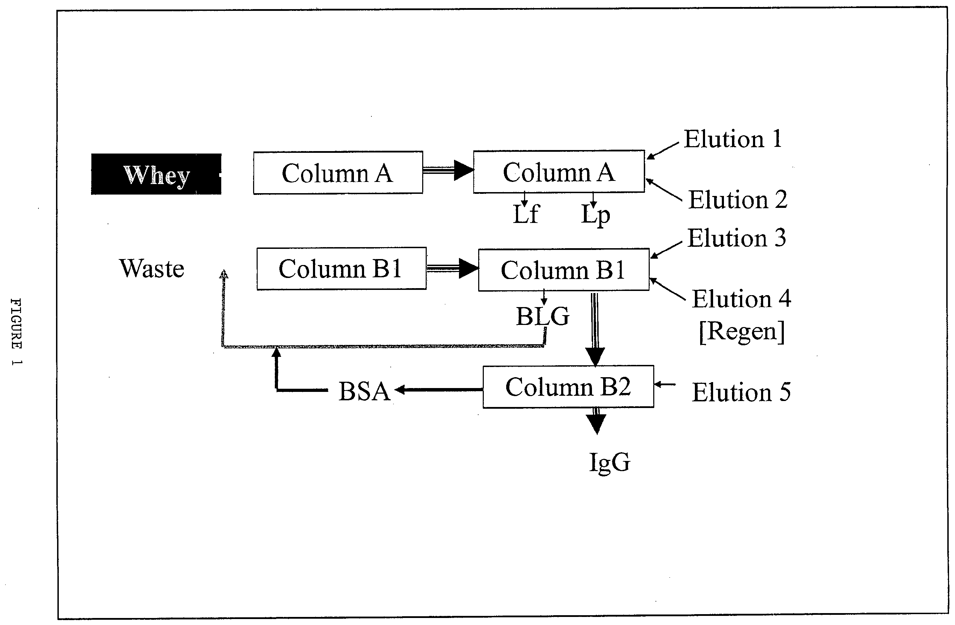

Flow chart in Figure 1 illustrates the overall process.
1. The liquid whey stream is first clarified via mechanical clarifier to remove any cheese fines solids
2. Column A is loaded with the clarified liquid whey stream then rinsed with water.
3. Elution with phosphate and chloride at pH 6 to 7 on Column A produces an eluant containing lactoperoxidase.
4. Elution with alkali at ambient temperature Column A produces an eluant containing lactoferrin. In turn, the pH of the lactoferrin eluate is adjusted to 7.
5. the pH of the run through from Column A (ie whey minus lactoperoxidase and lactoferrin) is adjusted to pH 4 to 5 with acid and loaded on to column B1.
6. The run through from step 5 is adjusted to a pH of 5 to 6 with alkali and reapplied to Column B1 to elute β-lactoglobulin.
7. Following a rinse of the column with water, alkali is applied to Column B1 to elute liquid immunoglobulins & Bovine Serum Albumin (BSA).
8. The pH of the liquid immunoglobulins and BSA fraction is adjusted to pH 4.5 to 5.0 with acid and loaded on to column B2.
9. BSA is trapped on Column B2 and the run through from Column B2 contains purified whey protein immunoglobulins removed for micro filtration step

Whey protein immunoglobulins are then subjected to ultrafiltration step to produce a concentrated 2% solids solution of purified whey protein immunoglobulins that can be freeze dried or spray dried (20% solids solution of purified whey protein immunoglobulins stored and transported into tanks at 0-4°C).

### Example 6 Immunoglobulin

Immunoglobulin may have the following composition:

| **Component** | **Amount** | **Method of determining amount** |
|---|---|---|
| Protein (% as is) | ≥ 90% | AS2300.1.2.1-(1991) |
| Immunoglobulins (%, IgG) | ≥ 30% | HPLC |
| Immunoglobulins (%, IgA) | ≥ 5% | ELISA |
| Fat | ≤ 1% | AS2300.1.3 (1998) |
| Moisture | ≤ 7% | AS2300.1.1 (1998) |
| Ash | ≤ 1.5% | AS2300.1.5 (1998) |

### Example 7 Lactoferrin

Lactoferrin may have the following composition:

| **Component** | **Amount** |
|---|---|
| Fat | < 1 % |
| Moisture | < 6 % |
| Ash | < 1.5 % |
| pH (1 % Solution) | 6 - 7 |
| Heavy metals: | ≤ 10 ppm |
| Protein (%, as is) | > 90 % |
| Lactoferrin (% of Protein, HPLC) | > 85 % |

Embodiments of the present invention are described in the following numbered paragraphs.
1. A composition including lactoferrin and immunoglobulin, wherein the composition does not substantially include one or more of the following proteins: lactoperoxidase, lactoglobulin, albumin.
2. The composition of paragraph 1 wherein the composition is provided in a form suitable for oral administration.
3. The composition of any one of the preceding paragraphs wherein the composition is provided in the form of a capsule or the like.
4. The composition of paragraph 3 wherein the immunoglobulin is provided in an amount of 25 to 35 (w/w%) of the capsule.
5. The composition of paragraph 4 wherein the lactoferrin may be provided in an amount of 60 to 70 (w/w%) of the capsule
6. The composition of any one of the preceding paragraphs wherein the composition is provided in the form of a liquid such as a syrup or the like.
7. The composition of paragraph 6 wherein the immunoglobulin is provided in an amount of 0.1 to 25 (w/w%) of the liquid.
8. The composition of paragraph 7 wherein the lactoferrin is provided in an amount of 0.1 to 25 (w/w%) of the liquid.
9. The composition of paragraph 1 wherein the composition is provided in a form suitable for topical administration.
10. The composition of paragraph 9 wherein the composition is provided in the form of a gel.
11. The composition of paragraph 10 wherein the immunoglobulin is provided in an amount of 0.1 to 25 (w/w%) of the gel.
12. The composition of paragraph 10 wherein the lactoferrin is provided in an amount of 0.1 to 25 (w/w%) of the gel.
13. The composition of any one of the preceding paragraphs wherein the composition is produced by a process including the steps of:
   -providing a source of lactoferrin;
   providing a source of immunoglobulin;
   combining said source of lactoferrin with said source of immunoglobulin, thereby producing the composition, wherein the source of immunoglobulin includes immunoglobulin in an amount of 40 to 95 (w/w%) of the source.
14. The composition of any one of the preceding paragraphs wherein at least part of the lactoferrin and immunoglobulin in the composition is obtained from a biological fluid or product derived from a biological fluid.
15. The composition of paragraph 14 wherein the biological fluid is milk or a related dairy product.
16. The composition of paragraph 15 wherein the fluid is whey stream.
17. The composition of any one of the preceding paragraphs wherein the immunoglobulin and/or lactoferrin in the composition is bovine.

## Claims

1. A composition consisting substantially of lactoferrin and immunoglobulin, wherein at least part of the lactoferrin and immunoglobulin in the composition is obtainable from a biological fluid or product derived from a biological fluid, and the biological fluid is a milk or related dairy product, wherein the lactoferrin and immunoglobulin are present in a mass ratio of 1:1, 2:1 or 5:1, and wherein the composition is orally administered.

2. The composition of claim 1 wherein lactoferrin and immunoglobulin are present in a mass ratio of 1:1.

3. The composition of any preceding claim wherein the composition is provided in the form of a capsule.

4. The composition of claim 3 wherein the immunoglobulin is provided in an amount of 25 to 35 (w/w%) of the capsule.

5. The composition of claim 4 wherein the lactoferrin is provided in an amount of 60 to 70 (w/w%) of the capsule.

6. The composition of claim 1 or 2 wherein the composition is provided in the form of a liquid.

7. The composition of claim 6 wherein the immunoglobulin is provided in an amount of 0.1 to 25 (w/w%) of the liquid.

8. The composition of claim 6 or 7 wherein the lactoferrin is provided in an amount of 0.1 to 25 (w/w%) of the liquid.

9. The composition of any one of the preceding claims wherein the composition is obtainable by a process including the steps of:
- providing a source of lactoferrin;
- providing a source of immunoglobulin;
combining said source of lactoferrin with said source of immunoglobulin, thereby producing the composition, wherein the source of immunoglobulin includes immunoglobulin in an amount of 40 to 95 (w/w%) of the source.

10. The composition of any of the preceding claims wherein the fluid is whey stream.

11. The composition of any one of the preceding claims wherein the immunoglobulin and/or lactoferrin in the composition is bovine.
